(19) Europäisches Patentamt
European Patent Office
Office européen des brevets

(11) Numéro de publication: **0 412 014 A1**

(12) # DEMANDE DE BREVET EUROPEEN

(21) Numéro de dépôt: **90402216.7**

(51) Int. Cl.5: **C07D 417/12**, A61K 31/54

(22) Date de dépôt: **02.08.90**

(30) Priorité: **04.08.89 FR 8910537**

(43) Date de publication de la demande: **06.02.91 Bulletin 91/06**

(84) Etats contractants désignés: **AT BE CH DE DK FR GB GR IT LI LU NL SE**

(71) Demandeur: **LABORATORIOS DEL DR. ESTEVE, S.A.**
**Av. Mare de Deu de Montserrat, 221**
**E-08026 Barcelona(ES)**

(72) Inventeur: **Ribalta-Baro, Jose Miguel, Ronda Canigo, 2;**
**casa No. 4, Urbanizacion "La Mayola"**
**E-08950 Esplugues de Llobregat(ES)**
Inventeur: **Frigola-Constansa, Jordi**
**Av. Diagonal, 299 at. 1a**
**E-08013 Barcelone(ES)**

(74) Mandataire: **Ahner, Francis et al**
**CABINET REGIMBEAU, 26, avenue Kléber**
**F-75116 Paris(FR)**

(54) **Dérivés benzothiaziniques, leur préparation et leurs applications comme médicaments ou comme intermédiaires de synthèse de médicaments.**

(57) L'invention concerne les composés répondant à la formule générale I

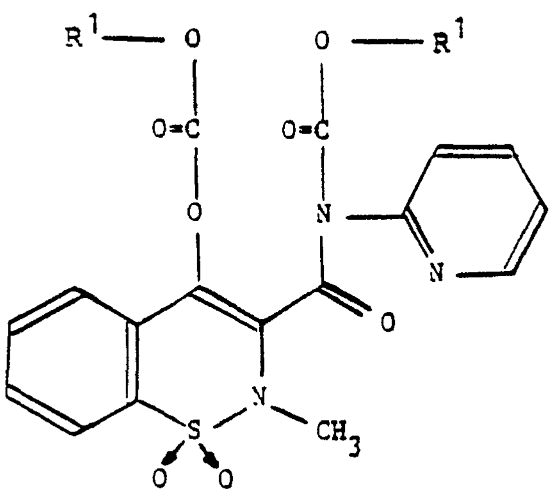

(I)

dans laquelle :
$R^1$ représente un radical alkyle inférieur (de un à quatre atomes de carbone), un radical phényle ou un radical benzyle. Ces composés sont utiles comme médicaments ou comme intermédiaires de synthèse de médicaments.

EP 0 412 014 A1

La présente invention concerne de nouveaux composés chimiques, répondant à la formule générale I, leur procédé de préparation, ainsi que leurs applications en tant que médicaments ou pour la préparation de médicaments :

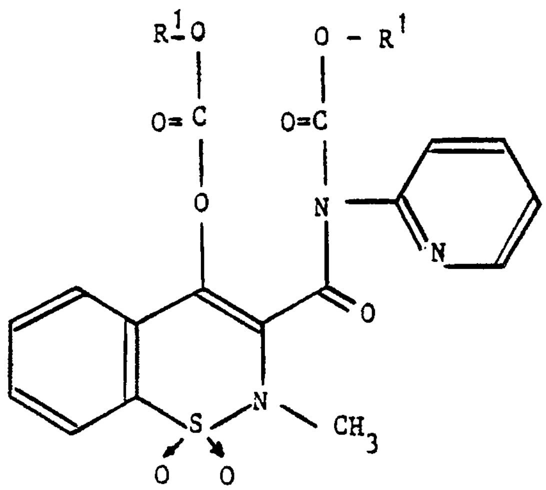

(I)

dans laquelle $R^1$ représente un radical alkyle inférieur en $C_1$ à $C_4$, un radical phényle ou un radical benzyle.

Les oxazinobenzothiazines 6,6-dioxyde, de formule générale II dans laquelle $R^2$ représente un radical hétéroaryle, sont des agents anti-inflammatoires et analgésiques bien connus, particulièrement le Droxicam ($R^2$ = 2-pyridyle), décrits dans Chem. Abst. , 1984, 100 , 191893j

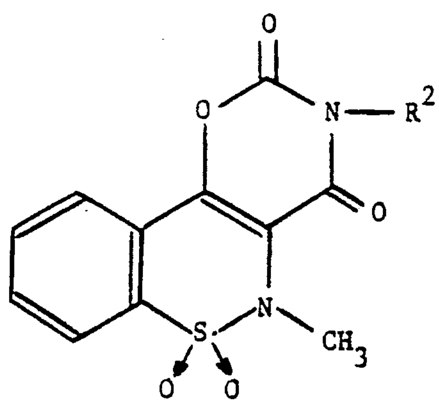

(II)

On a cherché à obtenir des intermédiaires simples et efficaces, qui permettent d'assurer la synthèse du Droxicam dans de bonnes conditions, et en particulier dans des conditions faciles à mettre en oeuvre à l'échelon industriel.

Dans la technique antérieure on préparait le Droxicam à partir d'un ester de formule (III)

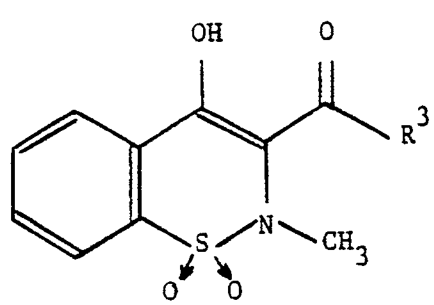

(III)

dans laquelle $R^3$ représente un radical alcoxy. En outre, les brevets FR-A-2 566 4508 et FR-2 597 103 décrivent la préparation du Droxicam à partir d'un composé de formule III, dans laquelle $R^3$ représente le radical 2-pyridylamino.

La présente invention concerne de nouveaux composés chimiques, répondant à la formule générale I, dans laquelle $R^1$ représente un radical alkyle inférieur (de un à quatre atomes de carbone), un radical phényle ou un radical benzyle.

Conformément à l'invention, on prépare les composés de formule I par réaction d'un composé de formule III, dans laquelle $R^3$ représente le radical 2-pyridylamino, avec un composé de formule générale IV,

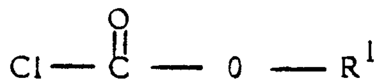

(IV)

dans laquelle $R^1$ a la signification donnée précédemment à propos de la formule générale I, en présence d'une base.

La réaction entre les composés de formules générales III et IV s'effectue à des températures comprises entre environ -5° C et environ 30° C, pendant un temps sensiblement compris entre 1 heure et 5 heures. Les quantités exactes des réactifs mis en présence ne sont pas critiques, mais il est toutefois préférable d'employer au moins deux proportions molaires du composé de formule IV pour chaque mole du composé de formule III, dans le but d'obtenir une réaction complète ainsi que de bons rendements.

La réaction s'effectue au sein d'un solvant organique choisi parmi la pyridine, la triéthylamine, les pyridines substituées telles que la 4-diméthylaminopyridine, la picoline, la lutidine, etc. ; le diméthyl sulfoxide, le dichlorométhane, le 1,2-dichloroéthane, le diméthylformamide, le 1,2-diméthoxyéthane, le dioxanne, le tétrahydrofuranne, etc. Dans le cas où le solvant utilisé n'est pas basique, il faut ajouter au moins deux équivalents molaires d'une base organique telle que pyridine, triéthylamine, triméthylamine, etc.

Alternativement on peut conduire la réaction entre les composés de formules III et IV en présence d'un hydrure de métal alcalin, tel que l'hydrure de sodium, au sein d'un solvant organique, par exemple dioxanne, tétrahydrofuranne, 1,2-diméthoxyéthane, toluène, xylènes ou solvants dipolaires aprotiques tels que diméthylsulfoxide, diméthylformamide, hexaméthylphosphotriamide, etc.

La réaction s'effectue aux mêmes températures et pendant le même temps décrits dans le cas où il s'agit d'une base organique.

En outre, les nouveaux composés chimiques de formule générale I, dans laquelle $R^1$ a la signification donnée ci-dessus, peuvent se transformer en Droxicam, de formule II dans laquelle $R^2$ représente le radical 2-pyridyle, par chauffage au sein d'un solvant organique tel que le pyridine, à des températures comprises entre 25 et 60° C pendant un temps compris entre 5 heures et 24 heures.

Ces composés de formule générale I trouvent donc leur application dans la préparation du Droxicam.

La réaction conduisant aux composés de formule générale I semble avoir lieu avec formation initiale d'un sel organique ou d'un métal alcalin, dont l'anion réagit avec un chloroformiate de formule IV. La formation postérieure d'un second anion et la nouvelle réaction avec un composé de formule IV donnerait lieu à la formation des composés objet de la présente demande, de formule générale I. La demanderesse n'entend toutefois pas être limitée par une telle interprétation.

La réaction peut se schématiser comme suit

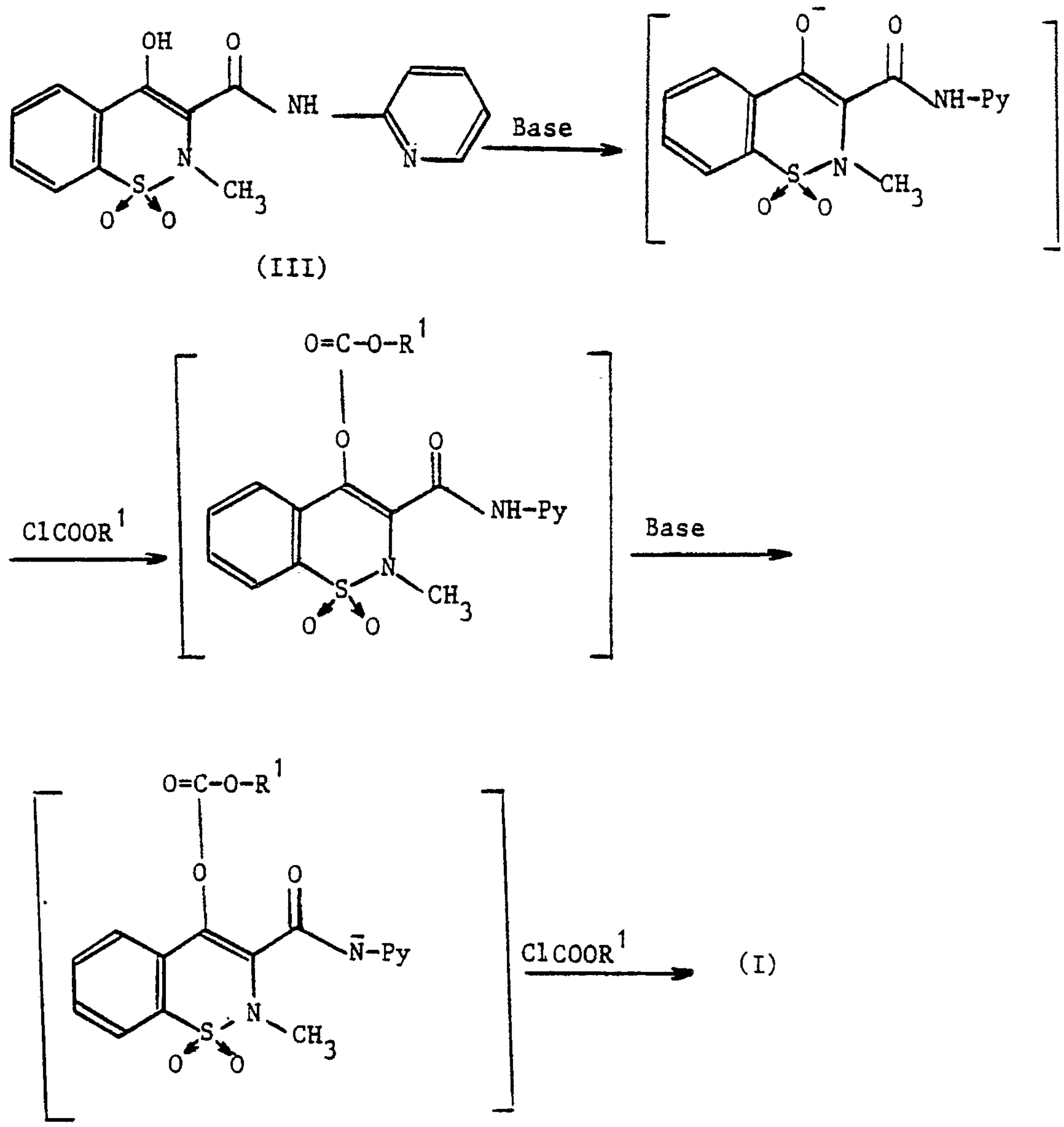


(III)

(I)

La présente invention s'applique donc également à l'utilisation des composés de formule générale I en tant qu'intermédiaires de synthèse de médicaments oxazinobenzothiaziniques, en particulier du Droxicam.

On décrira ci-après, à titre de simple exemple non limitatif, la préparation de dérivés de formule générale I et la formation de Droxicam, de formule générale II ($R^2$ = 2-pyridyl), à partir de dérivés de formule générale I.

## EXEMPLE 1

Préparation de N-éthoxycarbonyl-N-(2-pyridyl)-4-éthoxy-carbonyloxy-2-méthyl-2 H -1,2-benzothiazine-3-carboxamide 1,1-dioxyde

On ajoute lentement 90 ml (0,945 mole) de chloroformiate d'éthyle à une suspension de 75g (0,226 mole) de N-(2-pyridyl)-4-hydroxy-2-méthyl-2 H -1,2-benzothiazine-3-carboxamide 1,1-dioxyde dans 225 ml de pyridine anhydre à 0° C et on maintient la température en dessous de 10° C. On agite pendant une heure et on laisse le milieu réactionnel atteindre la température ambiante (environ 20° C). On verse la suspension sur 1 200 ml d'eau glacée, on agite une heure à 0-5° C, on filtre et on lave avec 200 ml d'eau froide.

On mélange sous agitation le produit brut avec 200 ml d'acétone froide, on filtre et on lave avec 100 ml d'acétone froide. On obtient 96,4g (rendement : 90%) d'un produit blanc de point de fusion 144-146° C. Par recristallisation avec de l'acétate d'éthyle, on obtient un solide blanc-jaunâtre de point de fusion 148-149° C.

Données spectroscopiques : 1H-RMN, , [DMSO-d6] :
1,10 (t,3H) ; 1,30 (t,3H) ; 3,30 (s,3H) ; 4,24 (q,2H) ; 4,35 (q,2H) ; 7,42 (m,2H) ; 7,60 (m, 1H) ; 7,80 (m, 2H) ; 7,96 (m,2H) ; 8,53 (d,1H)
IR(KBr) : 1755, 1748, 1690, 1335, 1245, 1015, 715 $cm^{-1}$.

## EXEMPLE 2

Préparation de N-éthoxycarbonyl-N-(2-pyridyl)-4-éthoxycarbonyloxy-2-méthyl-2 H -1,2-benzothiazine-3-carboxamide 1,1-dioxyde.

2,62 g d'hydrure de sodium à 55% en suspension dans de l'huile minérale sont lavés avec du n-pentane pour séparer l'huile par décantation. A l'hydrure de sodium résultant (1,44 g soit 0,06 mole) on ajoute 100 ml de tétrahydrofuranne et, dans un bain de glace à 5° C, on additionne 8,3 g (0,025 mole) de N-(2-pyridyl)-4-hydroxy-2-méthyl-2 H -1,2-benzothiazine-3-carboxamide 1,1-dioxyde. On agite pendant une heure en maintenant la température entre 0 et 5° C et on ajoute 6,7 ml (0,07 mole) de chloroformiate d'éthyle. On agite entre 1 et 2 heures jusqu'à atteindre la température ambiante (20° C), on filtre, on évapore le filtrat à sec, on lave à l'eau froide et on obtient 11,2 g (rendement : 94%). Par recristallisation avec de l'acétate d'éthyle, on obtient un produit de point de fusion 148-149° C, de caractéristiques spectroscopiques identiques à celles indiquées dans l'exemple 1.

## EXEMPLE 3

Dans des conditions d'opérations menées d'une façon similaire à celles décrites dans les exemples 1 et 2, les composés suivants peuvent être séparés en utilisant le chloroformiate, de formule générale IV, approprié.

- N-phénoxycarbonyl-N-(2-pyridyl)-4-phénoxycarboxyloxy-2-méthyl-2 H -1,2-benzothiazine-3-carboxamide 1,1-dioxyde ;
- N-Benzyloxycarbonyl-N-(2-pyridyl)-4-benzyloxycarboxyloxy-2-méthyl-2 H -1,2-benzothiazine-3-carboxamide 1,1-dioxyde ;
- N-méthoxycarbonyl-N-(2-pyridyl)-4-méthoxycarboxyloxy-2-méthyl-2 H -1,2-benzothiazine-3-carboxamide 1,1-dioxyde.

## EXEMPLE 4

Préparation de 5-méthyl-3-(2-pyridyl)-2 H , 5 H -1,3-oxazino [5,6-c ] [1,2]-benzothiazine-2,4-(3 H )-dione 6,6-dioxyde ; Droxicam.

On met en suspension 90 g (0,189 mole) de N-éthoxycarbonyl-N-(2-pyridyl)-4-éthoxycarbonyloxy-2-méthyl-2 H -1,2-benzothiazine-3-carboxamide 1,1-dioxyde dans 150 ml de pyridine anhydre. On chauffe le mélange à 35° C et on maintient pendant 15 heures cette température. On refroidit à température ambiante (20° C) et on verse sur 750 ml d'eau. On filtre le précipité obtenu, on le lave à l'eau et finalement avec de l'acétone.

On obtient ainsi 64 g (rendement : 95%). Par recristallisation avec du 1,2-dichloroéthane, on obtient un solide blanc cristallin de point de fusion 261-263° C.

Données spectroscopiques : 1H-RMN, $\delta$ , [DMSO-d6] :
3,02 (s,3H) ; 7,52 (m,2H) ; 7,92 (m,5H) ; 8,52 (d,1H)
IR (KBr) : 1185, 1355, 1410, 1640, 1710, 1790 $cm^{-1}$.

## ACTIVITE ANTI-INFLAMMATOIRE

(Inhibition de l'oedème produit par la carraghénine)

On a étudié l'activité anti-inflammatoire du composé de l'exemple 1 en déterminant l'inhibition de l'oedème induit par la carraghénine chez le rat Wistar et en le comparant avec la phénylbutazone. La méthode suivie a été celle décrite par Winter et cols. (Proc. Soc. Exp. Biol. Med. 1982 ; 111 ; 544-547).

On a calculé la DE-50 à partir des résultats obtenus 3 heures après l'administration des produits par voie orale, soit aussi 2 heures après l'injection de carraghénine à chaque patte (0,1 ml de solution de carraghénine à 2%, par voie sous-plantaire).

Le résumé des résultats obtenues est le suivant :

| Inhibition de l'oedème induit par carraghénine chez le rat Wistar 3 heures après l'administration du produit : | | | | |
|---|---|---|---|---|
| Produit | Dose (mg/kg, p.o.) | N | Inhibition oedème % | DE-50 (mg/kg) |
| Exemple 1 | 2,5<br>5<br>10<br>40 | 10<br>10<br>10<br>10 | 33,8<br>54,3<br>63,8<br>65,5 | 5,6 |
| Phénylbutazone | 3,12<br>12,5<br>25<br>50 | 10<br>10<br>10<br>10 | 21,5<br>46,6<br>52,1<br>63,5 | 19,6 |

On indiquera ci-après, à titre d'exemples, trois formes galéniques particulières des dérivés objet de la présente invention.

| Exemple de formule par gélule | |
|---|---|
| N-éthoxycarbonyl-N-(2-pyridyl)-4-éthoxycarbonyloxy-2 méthyl-2 H -1,2-benzothiazine-3-carboxamide-1,1-dioxyde | 0,020 g |
| Lactose | 0,136 g |
| Talc | 0,0016 g |
| Stéarate de magnésium | 0,0016 g |
| Aérosil-200 | 0,0008 g |
| Poids gélule | 0,160 g |

| Exemple de formule par comprimé | |
|---|---|
| N-éthoxycarbonyl-N-(2-pyridyl)-4-éthoxycarbonyloxy-2 méthyl-2 H -1,2-benzothiazine-3-carboxamide-1,1 dioxide | 0,020 g |
| Avicel pH 102 | 0,046 g |
| Lactose | 0,055 g |
| Primogel | 0,003 g |
| Polivinylpyrrolidone | 0,005 g |
| Stéarate de magnésium | 0,011 g |
| Poids comprimé | 0,100 g |

| Exemple de formule par suppositoire | |
|---|---|
| N-éthoxycarbonyl-N-(2-pyridyl)-4-éthoxycarbonyloxy-2-méthyl-2 H -1,2-benzothiazine-3-carboxamide-1,1-dioxyde | 0,050 g |
| Monolène | 1,950 g |
| Poids suppositoire | 2,000 g |

**Revendications**

1. Composés chimiques répondant à la formule générale I

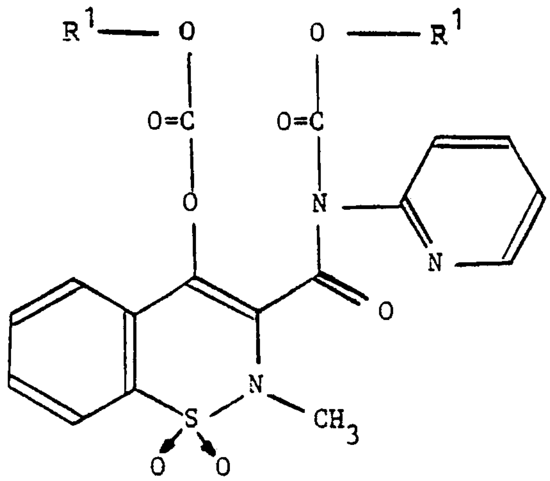

(I)

dans laquelle :
$R^1$ représente un radical alkyle inférieur (de un à quatre atomes de carbone), un radical phényle ou un radical benzyle.

2. Composé de formule générale I, selon la revendication 1, caractérisé en ce qu'il s'agit du N-éthoxycarbonyl N-(2-pyridyl)-4-éthoxycarbonyloxy-2-méthyl-2 H -1,2-benzothiazine-3-carboxamide-1,1-dioxyde.

3. Procédé de préparation des composés de formule générale I selon l'une des revendications 1 ou 2, caractérisé en ce que l'on fait réagir un composé de formule III

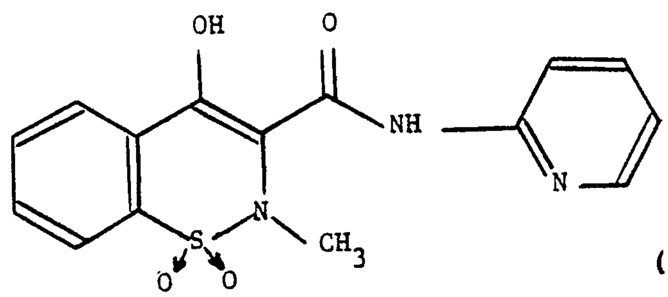

(III)

avec un composé de formule générale IV

$$Cl-\overset{\overset{O}{\|}}{C}-O-R^1 \qquad (IV)$$

dans laquelle :
$R^1$ représente un radical alkyle inférieur (de un à quatre atomes de carbone), un radical phényle ou un radical benzyle.

4. A titre de médicaments et/ou d'intermédiaires de synthèse de médicaments, les dérivés de formule générale I selon l'une des revendications 1 et 2.

5. Compositions pharmaceutiques caractérisées en ce qu'elles contiennent, outre un support pharmaceutiquement acceptable, un principe actif comprenant un dérivé de formule générale I selon l'une des

revendications 1 et 2.

Office européen des brevets

# RAPPORT DE RECHERCHE EUROPEENNE

Numéro de la demande

**EP 90 40 2216**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-3 900 470 (C.R. RASMUSSEN)<br>* En entier *<br>--- | 1,3-5 | C 07 D 417/12<br>A 61 K 31/54 |
| Y | US-A-3 925 371 (C.R. RASMUSSEN)<br>* En entier *<br>--- | 1,3-5 | |
| A | US-A-3 923 801 (C.R. RASMUSSEN)<br>* Colonnes 1-4 *<br>--- | 1,3 | |
| D,A | FR-A-2 597 103 (PROVESAN S.A.)<br>* Revendications *<br>----- | 1,3 | |

DOMAINES TECHNIQUES RECHERCHES (Int. Cl.5)

C 07 D 417/00
C 07 D 279/00
C 07 D 513/00
A 61 K 31/00

Le présent rapport de recherche a été établi pour toutes les revendications

| Lieu de la recherche | Date d'achèvement de la recherche | Examinateur |
|---|---|---|
| La Haye | 12 novembre 90 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant